# EUROPÄISCHE PATENTSCHRIFT

(11) **EP 1 556 131 B1**
(45) Veröffentlichungstag und Bekanntmachung des Hinweises auf die Patenterteilung: **22.11.2006**
(21) Anmeldenummer: 04731582.5
(22) Anmeldetag: 07.05.2004
(51) Int. Cl.: A61N 1/05, A61N 1/362

(54) **HERZSCHRITTMACHER-ELEKTRODENANORDNUNG**
PACEMAKER ELECTRODE ARRAY
SYSTEME D'ELECTRODE DE STIMULATEUR CARDIAQUE

(30) Priorität: 13.05.2003 DE 10321337
(43) Veröffentlichungstag der Anmeldung: 27.07.2005
(73) Patentinhaber: Peter Osypka Stiftung, 79639 Grenzach-Wyhlen (DE)
(72) Erfinder: OSYPKA, Peter, 79618 Rheinfelden-Herten (DE)
(74) Vertreter: Maucher, Wolfgang
(86) Internationale Anmeldenummer: PCT/EP2004/004864
(87) Internationale Veröffentlichungsnummer: WO 2004/101064

(56) Entgegenhaltungen:
- DE-C- 3 420 455
- US-A- 5 246 014
- US-A- 5 443 492

## Beschreibung

Die Erfindung betrifft eine Herzschrittmacher-Elektrodenanordnung mit einer in Gebrauchslage an der Außenseite eines Herzens oder von außen her am Herzen angreifenden und/oder mit einem Pol im Herzgewebe angeordneten oder fixierbaren und zu einem implantierten Herzschrittmacher verlaufenden Herzschrittmacherelektrode, die eine Elektrodenzuleitung aufweist und mit einem Anker von der Außenseite des Herzens her in Gebrauchsstellung festlegbar ist, und mit wenigstens einem zur Positionierung und Fixierung des Ankers dienenden Werkzeug und/oder Hilfsmittel, wobei als Werkzeuge oder Hilfsmittel zumindest eine Punktionskanüle und ein Einführschlauch für diese Punktionskanüle vorgesehen sind und der Innenquerschnitt des Einführschlauchs so groß bemessen ist, dass die Herzschrittmacherelektrode in ihn passt und in ihm verschiebbar ist.

Eine derartige Herzschrittmacher-Elektrodenanordnung ist aus US-A-5 443 492 bekannt.

Als Anker dient dabei eine etwa schraubenförmig verlaufende, über einen Teil des Umfangs der Elektrode reichende, an ihrem freien Ende angespitzte Windung, deren dem freien Ende entgegengesetzter Anfang an einem radial von der Elektrodenanordnung abstehenden Teil beginnt, wobei dieser Anker gegenüber dem distalen Ende der Elektrode einen Abstand hat.

Damit das distale Ende mit seinem Pol wirksam werden kann, muss es trotz dieses von ihm beabstandeten Ankers so fixiert werden, dass die Herzbewegungen den Kontakt nicht unterbrechen. Dabei können Schwierigkeiten vor allem daraus entstehen, dass an der Herzaußenseite häufig Fettgewebe vorhanden ist, welches den elektrischen Kontakt erschwert oder sogar ausschließt. Es ist deshalb erforderlich, das eigentliche, den Strom leitende distale Ende dieser bekannten Elektrodenanordnung in das Herzgewebe so tief einzubohren, dass der gegenüber dem distalen Ende zurückstehende Anker in diesem Gewebe fixiert werden kann. Um zu erkennen, ob die Elektrodenanordnung mit ihrem gegenüber dem distalen Ende beabstandeten Anker sicher fixiert ist, ist während der Implantation Sichtkontakt zum Herzen mit einer entsprechend großen Öffnung des Brustkorbes zweckmäßig.

Es besteht deshalb die Aufgabe, eine Elektrodenanordnung der eingangs genannten Art zu schaffen, bei welcher der Brustkorb kaum geöffnet werden muss, sondern ein minimalinvasiver Zugriff zum Herzen ausreicht, dennoch die Elektrode aber sicher im Myokard fixiert werden kann, ohne auf eine äußere Fettschicht am Herzen Rücksicht nehmen zu müssen. Gleichzeitig soll der Platzbedarf für den Angriff der Elektrode an der Herzaußenseite möglichst gering sein, während gleichzeitig die Reizleitung möglichst gut sein soll.

Diese Aufgabe wird mit den Mitteln und Merkmalen einerseits des Patentanspruchs 1 und andererseits des Patentanspruchs 2 gelöst.

Eine solche Herzschrittmacher-Elektrodenanordnung ermöglicht es, unabhängig von einer an der Herzaußenseite befindlichen Fettschicht den Anker und damit das Ende der Elektrode im Inneren des Myokards zu platzieren, so dass eine gute Fixation und außerdem eine bestmögliche Reizleitung erzielt werden. Dabei kann in vorteilhafter Weise eine nur geringe Öffnung am Brustkorb ausreichen, um die Elektrode am Herzen verankern zu können, weil in vorteilhafter Weise eine zur Vorbereitung der Verankerung einsetzbare Punktionskanüle mit einem Einführschlauch an der entsprechenden Stelle des Herzens eingesetzt und dann aus diesem Schlauch in bekannter Weise entfernt werden kann, wonach die eigentliche Elektrode durch diesen Schlauch in die vorbereitete Öffnung am Herzen eingeschoben und verankert werden kann.

Die Herzschrittmacherelektrode kann also nach dem Zurückziehen der Punktionskanüle durch den von der Außenseite her in das Herzgewebe führenden Einführschlauch in das Herzgewebe und in den von der Punktionskanüle darin gebildeten Kanal einschiebbar sein. Somit kann in platzsparender Weise mit einem kleinen Anker dennoch effektiv eine Befestigung im Myokard bewirkt werden, ohne dass eine außenseitige Fettschicht diese Verankerung behindert oder gar zu einer unzureichenden Verankerung führt.

Durch die erfindungsgemäße Lösung gemäß Patentanspruch 1 ist es möglich, die Herzschrittmacherelektrode mit ihrer als Anker ausgebildeten, in Fortsetzung ihrer Längsmittelachse angeordneten Schraubwendel entgegen der Schraubrichtung zu verdrehen und zu tordieren und in dieser Position mit Hilfe des Stiletts festzuhalten, dann einzuführen und schließlich innerhalb des Myokards in die entspannte Position zu verdrehen und dadurch die Schraubwendel auch im Myokard einzuschrauben und zu verankern. Danach kann in üblicher Weise der Einführschlauch entfernt werden, gegebenenfalls unter Auftrennung einer Längsseite, wie es beispielsweise auch bei Einführhülsen gemäß DE 34 20 455 C1 in analoger Weise bekannt ist.

Mit der erfindungsgemäßen Lösung gemäß Patentanspruch 2 ist es möglich, statt einer Schraubwendel als Anker widerhakenartig wirkende Vorsprünge vorzusehen, die vor allem nach dem Einführen durch den Einführschlauch eine selbsttätige Verankerung im Myokard ergeben. Ferner ist es möglich, bei einer Elektrodenanordnung mit einem als Schraubwendel ausgebildeten Anker zusätzlich derartige widerhakenartig wirkende Vorsprünge als einen oder mehrere weitere Anker zu verwenden, um die Befestigung im Myokard noch zu verbessern.

Vor allem können auch in axialer Richtung mehrere Anker hintereinander vorgesehen sein, um wiederum die Festlegung am Herzen zu verbessern.

Die Elektrodenanordnung kann biventrikulär ausgebildet sein und eine gemeinsame Zuleitung kann zwei sich verzweigende und dann getrennt bis zum Herzen verlaufende Elektroden aufweisen, deren jede wenigstens einen Anker zum Befestigen am Herzen hat. Dabei kann die gemeinsame Zuleitung von einem Steuergerät beziehungsweise von dem Herzschrittmacher für die Elektroden so nah wie möglich an das Herz führen, so dass die verzweigten Elektroden entsprechend kurz sind und einfach durch den Brustkorb zum Herzen geführt werden können, um dort in Gebrauchsstellung entsprechende Reize weiterleiten zu können.

Die Anode der Elektrode oder der Elektroden kann außerhalb des Herzens mit Abstand zu der Katode beziehungsweise zu dem am Herzen befindlichen Pol und bei einer biventrikulären verzweigten Elektrode im Bereich der gemeinsamen Zuleitung vor der Verzweigung angeordnet sein. Dadurch kann der Querschnitt in dem Myokardkanal möglichst klein und die Dauerbelastung des Herzens durch die an ihm verankerte Elektrodenanordnung geringer gehalten werden.

Vor allem bei Kombination einzelner oder mehrerer der vorbeschriebenen Merkmale und Maßnahmen ergibt sich eine Elektrodenanordnung, die platzsparend und bei guter Reizleitung dennoch dauerhaft an der Außenseite des Herzens im Myokard verankert werden kann, ohne dass es dafür einer großen Öffnung am Brustkorb bedarf.

Nachstehend sind Ausführungsbeispiele der Erfindung anhand der Zeichnung näher beschrieben. Es zeigt in zum Teil schematisierter Darstellung:
- Fig. 1: eine erfindungsgemäße Herzschrittmacher-Elektrodenanordnung, die biventrikulär ausgebildet ist, in Gebrauchslage,
- Fig. 2: in vergrößertem Maßstab die Anordnung einer zu der erfindungsgemäßen Herzschrittmacher-Elektrodenanordnung gehörenden Punktionskanüle und eines Einführschlauchs in deren Gebrauchslage vor dem Verankern der Herzschrittmacherelektrode,
- Fig. 3: eine biventrikulär ausgebildete Herzschrittmacherelektrode, deren eine Elektrode bereits im Myokard verankert ist, während die andere sich noch in dem Einführschlauch befindet, aus welchem die Punktionskanüle zuvor entfernt wurde, wobei als Anker in Verlängerung der Elektroden jeweils eine Schraubwendel vorgesehen ist,
- Fig. 4: eine Ansicht einer biventikulären Herzschrittmacherelektrode, an deren beiden Elektroden in axialer Richtung hintereinander angeordnete ringförmige Vorsprünge als Anker vorgesehen sind,
- Fig. 5: eine gegenüber Fig.4 abgewandelte Ausführungsform mit an jeder Elektrode widerhakenartig abstehenden stiftförmigen Vorsprüngen,
- Fig. 6: eine wiederum angewandelte Ausführungsform, bei welcher an den beiden distalen Enden der Elektroden jeweils ein Widerhaken als Anker vorgesehen ist,
- Fig. 7: die Verankerung des distalen Endes einer zu einer erfindungsgemäßen Elektrodenanordnung gehörenden Elektrode mit Hilfe von widerhakenartigen Vorsprüngen, die in Einführrichtung der Elektrode hinter dem distalen Pol angeordnet sind,
- Fig. 8: eine der Fig.7 entsprechende Anordnung, bei welcher widerhakenartig angeordnete stiftförmige Vorsprünge selbst unmittelbar am distalen Ende der Elektrode von dieser schräg nach hinten abstehen, sowie
- Fig. 9: die Darstellung einer im Myokard verankerten Elektrode, die an ihrem distalen Ende eine enge Schraubwendel als Anker aufweist.

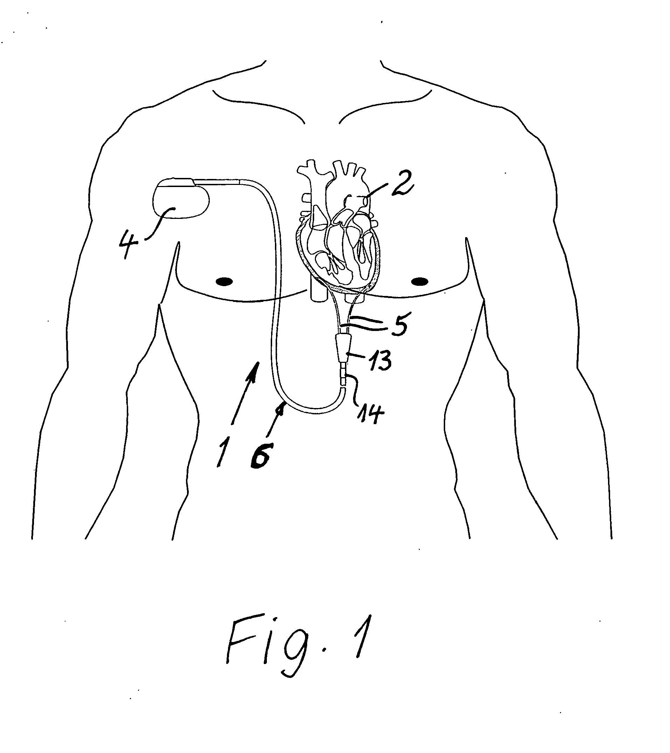

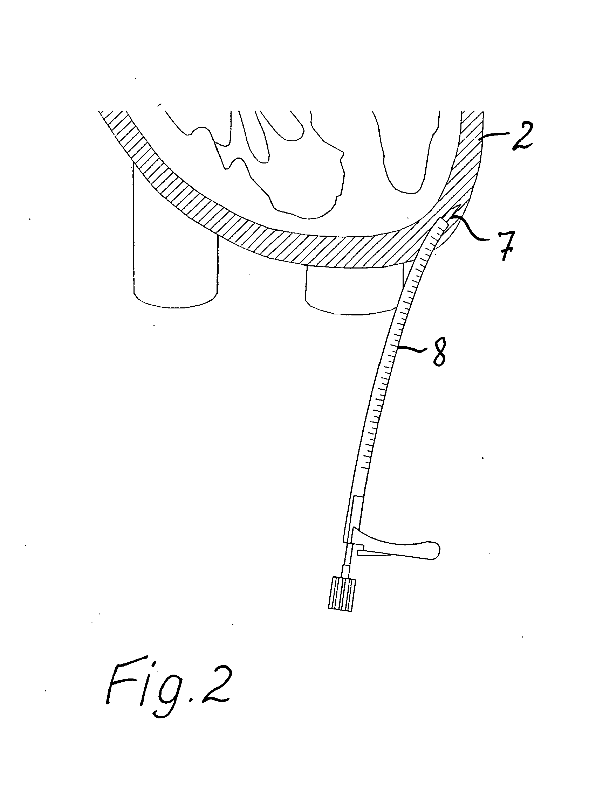

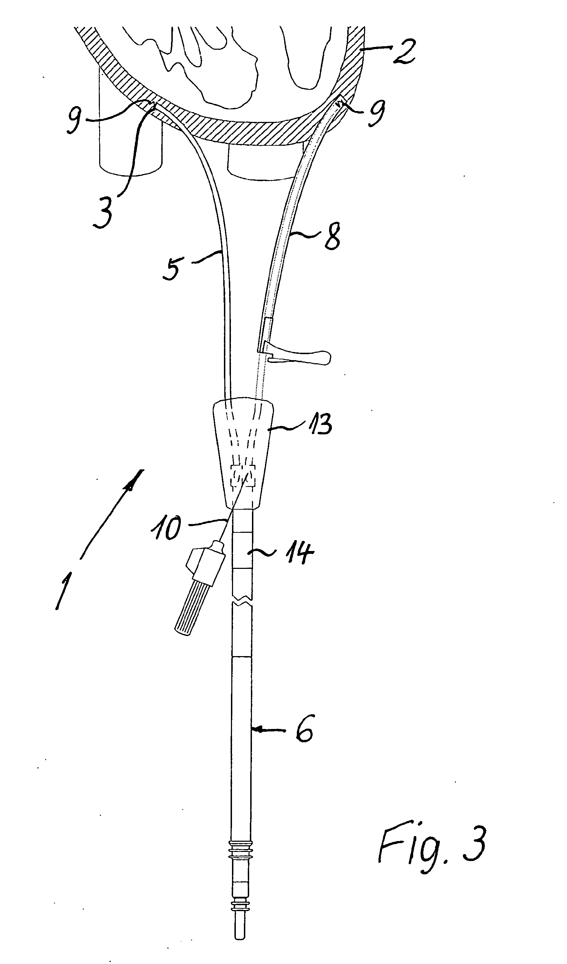

Eine im Ganzen mit 1 bezeichnete Herzschrittmacher-Elektrodenanordnung, die in den Zeichnungen besonders gut bei gemeinsamer Betrachtung der Fig.1 und 3 erkennbar ist, weist wenigstens eine, im dargestellten Ausführungsbeispiel zwei in Gebrauchslage an der Außenseite eines Herzens 2 oder von außen her am Herzen 2 angreifende und mit einem Pol 3 im Herzgewebe angeordnete und zu einem implantierbaren Herzschrittmacher 4 verlaufende Herzschrittmacherelektroden 5 auf. Ferner gehört zu dieser Elektrodenanordnung 1 eine Elektrodenzuleitung 6 und ein im folgenden noch näher zu beschreibender, bei den einzelnen Ausführungsbeispielen unterschiedlich gestalteter und deshalb jeweils mit eigener Bezugszahl versehener Anker, womit die jeweilige Herzschrittmacherelektrode 5 beziehungsweise ihr Pol 3 von der Außenseite des Herzens 2 her im Myokard festlegbar ist. Ferner gehört zu der Elektrodenanordnung 1 ein oder mehrere Werkzeuge und/oder Hilfsmittel, die vor allem in den Fig. 2 und 3 dargestellt und nachstehend erläutert sind.

Als Werkzeug oder Hilfsmittel ist zumindest eine Punktionskanüle 7 (Fig.2) und ein Einführschlauch 8 für die Punktionskanüle 7 vorgesehen. Dabei ist der Innenquerschnitt des Einführschlauchs 8 so groß bemessen, dass die Herzschrittmacherelektrode 5 mit ihrem am distalen Ende vorgesehenen Anker, beispielsweise einer nachstehend näher erläuterten Schraubwendel 9, in diesen Einführschlauch passt und in ihm verschiebbar ist. Vor allem beim Vergleich der Fig.2 und 3 erkennt man, dass zunächst die Punktionskanüle 7 mit dem Einführschlauch 8 benutzt wird, wobei die Kanüle 7 über den Schlauch 8 übersteht und im Myokard einen entsprechenden Kanal erzeugt. Anschließend kann die Kanüle 7 zurückgezogen werden. Die Herzschrittmacherelektrode ist dann nach dem Zurückziehen der Funktionskanüle 7 durch den von der Außenseite her in das Herzgewebe führenden Einführschlauch 8 in das Herzgewebe und in den von der Funktionskanüle 7 darin erzeugten Kanal einschiebbar, wie es in Fig.3 deutlich erkennbar ist.

Die in diesem Ausführungsbeispiel als Anker dienende, am distalen Ende der Herzschrittmacherelektrode 5 angeordnete Schraubwendel 9 setzt dabei mit ihrer Mittelachse die Längsmittelachse dieser Herzschrittmacherelektrode 5 fort, befindet sich also mit dieser in Flucht und kann einen übereinstimmenden oder eventuell sogar etwas kleineren oder gegebenenfalls auch etwas größeren Außendurchmesser als die eigentliche Herzschrittmacherelektrode 5 haben.

Dabei ist diese Herzschrittmacher-Elektrode 5 in an sich üblicher und bekannter Weise derart flexibel, dass sie in sich verdrehbar beziehungsweise tordierbar ist. In ihr verläuft dabei ein Kanal, in welchen ein als weiteres Werkzeug dienendes Stilett 10 passt, das im Bereich der Schraubwendel 9 mit seinem Arbeitsende zu einer dort vorgesehenen Profilierung, beispielsweise einer abgeflachten Form oder Ausnehmung, passt, so dass mit diesem Stilett 10 die Verdrehung der Schraubwendel 9 zunächst in eine tordierte Lage und vor allem nach dem Einführen in das Myokard in eine entspannte und zurückverdrehte Lage möglich ist, wodurch die Schraubwendel 9 und damit die Herzschrittmacherelektrode 5 verankert werden.

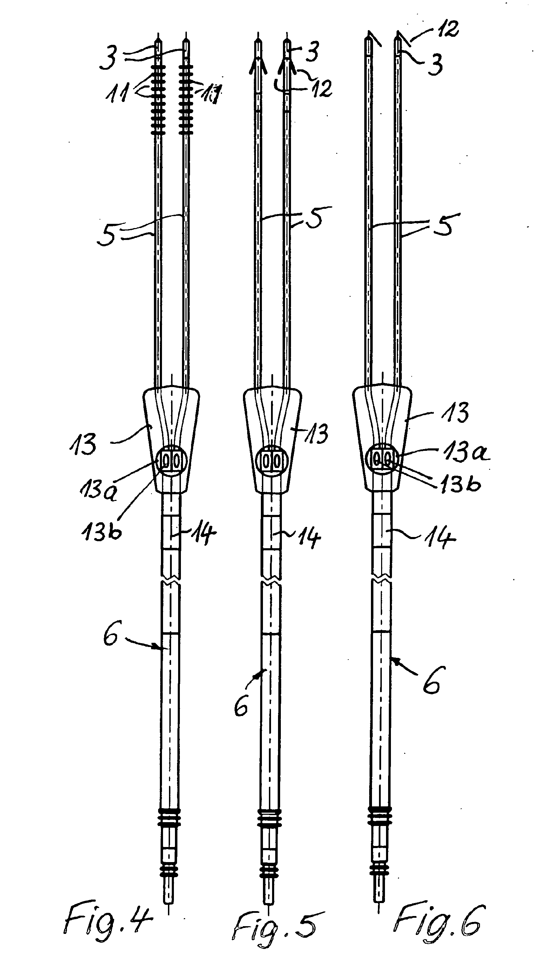

In den Ausführungsbeispielen gemäß Fig.4 bis 6 sind am distalen Ende der Herzschrittmacherelektroden 5 ringartige Vorsprünge 11, gegebenenfalls auch schirmartige derartige Vorsprünge oder widerhakenartige stiftförmige Vorsprünge 12 als Anker angeordnet, wobei diese stiftartigen Vorsprünge 12 gemäß Fig.6 einzeln oder gemäß Fig.5 paarweise und dabei auch an etwas unterschiedlichen Stellen der jeweiligen Herzschrittmacherelektrode 5, nämlich ganz am Ende oder mit etwas Abstand zum Ende vorgesehen sein können. Die ringförmigen Vorsprünge 11 sind dabei in axialer Richtung als mehrere Anker hintereinander angeordnet.

In den Ausführungsbeispielen gemäß Fig.1 und 3 bis 6 ist die Elektrodenanordnung 1 biventrikulär ausgebildet, dass heißt an einer gemeinsamen Zuleitung 6, die von dem Herzschrittmacher 4 ausgehend, sind zwei sich verzweigende und dann getrennt bis zum Herzen 2 verlaufende Elektroden 5 vorgesehen, deren jede wenigstens einen der beschriebenen Anker 9, 11 und/oder 12 zum Befestigen am Herzen 2 hat. Stattdessen könnte jedoch auch ohne eine Verzweigung 13 nur eine Zuleitung 6 vorgesehen sein, die sich als Elektrode 5 bis zu ihrem Anker ihm Herzen fortsetzt.

Die Verzweigung 13 hat eine Öffnung 13a zu Eintritten 13b für das Stilett 10, übt also eine Doppelfunktion aus.

In den Figuren erkennt man außerdem noch, dass die Anode 14 der Elektrode oder Elektroden 5 außerhalb des Herzens 2 mit Abstand zu der Katode beziehungsweise zu dem am Herzen befindlichen Pol 3 und dabei bei einer biventrikulären verzweigten Elektrode im Bereich der gemeinsamen Zuleitung 6 vor der Verzweigung 13 angeordnet ist, so dass die eigentlichen Elektroden 5 entsprechend dünner ausgestaltet sein können.

Die Herzschrittmacher-Elektrodenanordnung 1 mit einer an der Außenseite eines Herzens 2 angreifenden und mit einem Pol 3 in Gebrauchsstellung im Herzgewebe angeordneten und zu einem implantierten Herzschrittmacher 4 verlaufenden Herzschrittmacherelektrode 5 weist als ihr zugehöriges Hilfsmittel eine Punktionskanüle 7 und einen Einführschlauch 8 für diese Punktionskanüle 7 auf, dessen Innenquerschnitt so groß bemessen ist, dass die Herzschrittmacherelektrode 5 nach dem Zurückziehen der Punktionskanüle 7 in diesen Einführschlauch 8 passt und in ihm bis in das Herzgewebe einschiebbar ist. Danach kann der Einführschlauch 8 in bekannter Weise zurückgezogen oder durch einen seitliche Öffnung zur Seite hin abgezogen werden.

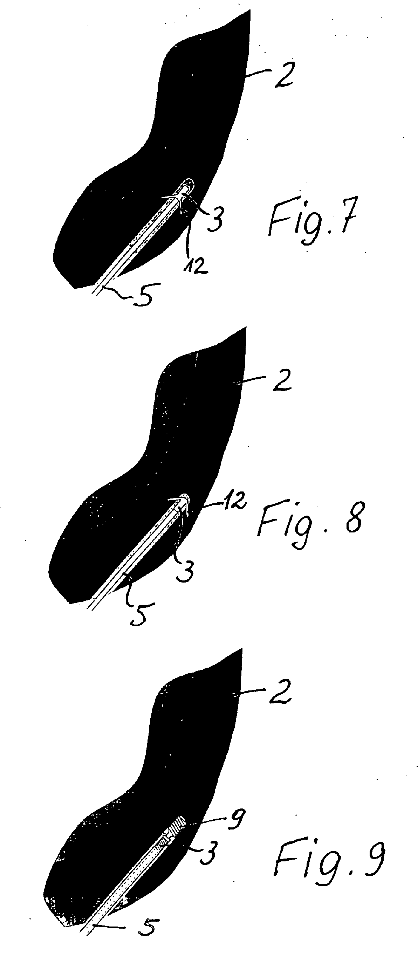

In den Fig.7, 8 und 9 ist noch die verankerte Gebrauchsstellung des distalen Endes von Herzschrittmacherelektroden 5 dargestellt, die unterschiedlich ausgebildete widerhakenartige Anker (Fig. 7 und 8) oder eine Schraubwendel 9 als Anker haben, wobei diese Schraubwendel 9 gemäß Fig.9 von engen Windungen gebildet ist, die sich innerhalb des im Myokard befindlichen, von der Punktionskanüle 7 geschaffenen Kanals verklemmen und einwachsen können.

## Patentansprüche

1. Herzschrittmacher-Elektrodenanordnung (1) mit einer in Gebrauchslage an der Außenseite eines Herzens (2) oder von außen her am Herzen (2) angreifenden und/oder mit einem Pol (3) im Herzgewebe angeordneten oder fixierbaren und zu einem implantierten Herzschrittmacher (4) verlaufenden Herzschrittmacherelektrode (5), die eine Elektrodenzuleitung (6) aufweist und mit einem Anker von der Außenseite des Herzens (2) her in Gebrauchsstellung festlegbar ist, und mit wenigstens einem zur Positionierung und/oder Fixierung des Ankers dienenden Werkzeug und/oder Hilfsmittel, wobei als Werkzeuge oder Hilfsmittel zumindest eine Punktionskanüle (7) und ein Einführschlauch (8) für diese Punktionskanüle (7) vorgesehen sind und der Innenquerschnitt des Einführschlauchs (8) so groß bemessen ist, dass die Herzschrittmacherelektrode in ihn passt und in ihm verschiebbar ist, wobei der Anker am distalen Ende der Herzschrittmacherelektrode (5) angeordnet ist und diese mit dem Anker in den Einführschlauch (8) passt und in ihm verschiebbar ist, wobei an dem distalen Ende der Herzschrittmacherelektrode (5) als Anker eine Schraubwendel (9) angeordnet ist, deren Mittelachse die Fortsetzung der Längsmittelachse der Herzschrittmacherelektrode (5) bildet, wobei die Herzschrittmacherelektrode (5) derart flexibel ist, dass sie tordierbar ist, und wobei in ihr ein Kanal für ein als Werkzeug oder als weiteres Werkzeug dienendes Stilett (10) und im Bereich der Schraubwendel (9) eine Profilierung oder eine abgeflachte Form oder Ausnehmung angeordnet ist, die mit dem Arbeitsende des Stiletts (10) oder Werkzeugs in Drehrichtung formschlüssig zusammenpasst.

2. Herzschrittmacher-Elektrodenanordnung (1) mit einer in Gebrauchslage an der Außenseite eines Herzens (2) oder von außen her am Herzen (2) angreifenden und/oder mit einem Pol (3) im Herzgewebe angeordneten oder fixierbaren und zu einem implantierten Herzschrittmacher (4) verlaufenden Herzschrittmacherelektrode (5), die eine Elektrodenzuleitung (6) aufweist und mit einem Anker von der Außenseite des Herzens (2) her in Gebrauchsstellung festlegbar ist, und mit wenigstens einem zur Positionierung und/oder Fixierung des Ankers dienenden Werkzeug und/oder Hilfsmittel, wobei als Werkzeuge oder Hilfsmittel zumindest eine Punktionskanüle (7) und ein Einführschlauch (8) für diese Punktionskanüle (7) vorgesehen sind und der Innenquerschnitt des Einführschlauchs (8) so groß bemessen ist, dass die Herzschrittmacherelektrode in ihn passt und in ihm verschiebbar ist, wobei der Anker am distalen Ende der Herzschrittmacherelektrode (5) angeordnet ist und diese mit dem Anker in den Einführschlauch (8) passt und in ihm verschiebbar ist und wobei am distalen Ende der Herzschrittmacherelektrode (5) wenigstens ein ringartiger Vorsprung (11), schirmartiger Vorsprung und/oder widerhakenartiger Vorsprung (12) als Anker oder zusätzlicher Anker angeordnet ist.

3. Elektrodenanordnung nach Anspruch 1 oder 2, **dadurch gekennzeichnet, dass** die Herzschrittmacherelektrode (5) nach dem Zurückziehen der Punktionskanüle (7) durch den von der Außenseite her in das Herzgewerbe führenden Einführschlauch (8) in das Herzgewebe und in den von der Punktionskanüle (7) darin gebildeten Kanal einschiebbar ist.

4. Elektrodenanordnung nach einem der Ansprüche 1 bis 3, **dadurch gekennzeichnet, dass** in axialer Richtung mehrere Anker (9, 11, 12) hintereinander vorgesehen sind.

5. Elektrodenanordnung nach einem der Ansprüche 1 bis 4, **dadurch gekennzeichnet, dass** sie biventrikulär ausgebildet ist und dass eine gemeinsame Zuleitung (6) zwei sich verzweigende und dann getrennt bis zum Herzen (2) verlaufende Elektroden (5) aufweist, deren jede wenigstens einen Anker zum Befestigen am Herzen (2) hat.

6. Elektrodenanordnung nach einem der Ansprüche 1 bis 5, **dadurch gekennzeichnet, dass** die Anode (14) der Elektrode oder Elektroden (5) außerhalb des Herzens (2) mit Abstand zu der Katode beziehungsweise zu dem am Herzen (2) befindlichen Pol (3) und bei einer biventrikulären verzweigten Elektrodenanordnung im Bereich der gemeinsamen Zuleitung (6) vor der Verzweigung (13) angeordnet ist/sind.

## Claims

1. Pacemaker electrode array (1) having a pacemaker electrode (5) which in the position of use acts on the outside of a heart (2) or acts on the heart (2) from the outside and/or is arranged or fixable in the heart tissue with a pole (3) and leads to an implanted pacemaker (4), said electrode (5) having an electrode lead wire (6) and being fastenable in the position of use by means of an anchor from outside the heart (2), and with at least one tool and/or aid serving to position and/or fix the anchor, the tools or aids provided being at least one puncture cannula (7) and an insertion tube (8) for this puncture cannula (7) and the inner cross-section of the insertion tube (8) being of such dimensions that the pacemaker electrode fits into it and is movable therein, the anchor being disposed at the distal end of the pacemaker electrode (5) and this electrode (5) together with the anchor fitting into the insertion tube (8) and being movable therein, while at the distal end of the pacemaker electrode (5) a helix (9) is provided as the anchor, its central axis forming a continuation of the central longitudinal axis of the pacemaker electrode (5), the pacemaker electrode (5) being flexible to such an extent that it is twistable, and wherein a channel is provided therein for a stylet (10) serving as a tool or as a further tool and in the region of the helix (9) a profiling or flattened shape or recess is provided which fits by interlocking engagement with the working end of the stylet (10) or tool in the direction of rotation.

2. Pacemaker electrode array (1) having a pacemaker electrode (5) which in the position of use acts on the outside of a heart (2) or acts on the heart (2) from the outside and/or is arranged or fixable in the heart tissue with a pole (3) and leads to an implanted pacemaker (4), said electrode (5) having an electrode lead wire (6) and being fastenable in the position of use by means of an anchor from outside the heart (2), and with at least one tool and/or aid serving to position and/or fix the anchor, the tools or aids provided being at least one puncture cannula (7) and an insertion tube (8) for this puncture cannula (7) and the inner cross-section of the insertion tube (8) being of such dimensions that the pacemaker electrode fits into it and is movable therein, the anchor being disposed at the distal end of the pacemaker electrode (5) and this electrode (5), together with the anchor, fitting into the insertion tube (8) and being movable therein, and wherein at the distal end of the pacemaker electrode (5) at least one annular projection (11), umbrella-like projection and/or barb-like projection (12) is provided as an anchor or additional anchor.

3. Electrode array according to claim 1 or 2, **characterised in that** after the withdrawal of the puncture cannula (7) through the insertion tube (8) leading into the heart tissue from the outside, the pacemaker electrode can be inserted into the heart tissue and into the channel formed therein by the puncture cannula (7).

4. Electrode array according to one of claims 1 to 3, **characterised in that** a plurality of anchors (9, 11, 12) are provided behind one another in the axial direction.

5. Electrode array according to one of claims 1 to 4, **characterised in that** it is of biventricular construction and that a common lead wire (6) has two electrodes that branch off and then run separately to the heart (2), each having at least one anchor for attachment to the heart (2).

6. Electrode array according to one of claims 1 to 5, **characterised in that** the anode(s) (14) of the electrode or electrodes (5) is or are arranged outside the heart (2) at a spacing from the cathode or from the pole (3) situated on the heart (2) and in a biventricular branched electrode arrangement is or are arranged in the region of the common lead wire (6) in front of the branching point (13).

## Revendications

1. Système d'électrode de stimulateur cardiaque (1) avec une électrode de stimulateur cardiaque (5) agissant en position d'utilisation sur l'extérieur d'un coeur (2) ou sur le coeur (2) depuis l'extérieur et/ou disposée ou pouvant être fixée dans le tissu cardiaque par un pôle (3) et allant à un stimulateur cardiaque (4) implanté, qui présente une ligne d'alimentation d'électrode (6) et peut être fixée en position d'utilisation depuis l'extérieur du coeur (2) avec une ancre, et avec au moins un outil et/ou accessoire servant au positionnement et/ou à la fixation de l'ancre, sachant qu'au moins une canule de ponction (7) et un tuyau d'introduction (8) pour cette canule de ponction (7) sont prévus comme outils ou accessoires et que la section intérieure du tuyau d'introduction (8) est d'une dimension suffisante pour que l'électrode de stimulateur cardiaque puisse passer et être déplacée dedans, sachant que l'ancre est disposée à l'extrémité distale de l'électrode de stimulateur cardiaque (5) et que cette dernière peut, avec l'ancre, passer dans le tuyau d'introduction (8) et être déplacée dans celui-ci, sachant qu'une spirale de fixation (9), dont l'axe médian forme le prolongement de l'axe médian longitudinal de l'électrode de stimulateur cardiaque (5), est disposée à l'extrémité distale de l'électrode de stimulateur cardiaque (5) en guise d'ancre, sachant que l'électrode de stimulateur cardiaque (5) est assez flexible pour pouvoir être pliée, et sachant qu'un canal pour un stylet (10) servant d'outil ou d'outil auxiliaire passe à l'intérieur de celle-ci et qu'un profilage ou une forme ou un évidement aplati(e) qui s'adapte par engagement positif en sens de rotation avec l'extrémité de travail du stylet (10) ou outil est disposé(e) au niveau de la spirale de fixation (9).

2. Système d'électrode de stimulateur cardiaque (1) avec une électrode de stimulateur cardiaque (5) agissant en position d'utilisation sur l'extérieur d'un coeur (2) ou sur le coeur (2) depuis l'extérieur et/ou disposée ou pouvant être fixée dans le tissu cardiaque par un pôle (3) et allant à un stimulateur cardiaque (4) implanté, qui présente une ligne d'alimentation d'électrode (6) et peut être fixée en position d'utilisation depuis l'extérieur du coeur (2) avec une ancre, et avec au moins un outil et/ou accessoire servant au positionnement et/ou à la fixation de l'ancre, sachant qu'au moins une canule de ponction (7) et un tuyau d'introduction (8) pour cette canule de ponction (7) sont prévus comme outils ou accessoires et que la section intérieure du tuyau d'introduction (8) est d'une dimension suffisante pour que l'électrode de stimulateur cardiaque puisse passer et être déplacée dedans, sachant que l'ancre est disposée à l'extrémité distale de l'électrode de stimulateur cardiaque (5) et que cette dernière peut, avec l'ancre, passer dans le tuyau d'introduction (8) et être déplacée dans celui-ci et sachant qu'au moins une saillie (11) de type anneau, une saillie de type parapluie et/ou une saillie de type barbillon (12) est disposée en guise d'ancre ou d'ancre supplémentaire à l'extrémité distale de l'électrode de stimulateur cardiaque (5).

3. Système d'électrode selon la revendication 1 ou 2, **caractérisé par le fait qu'**après le retrait de la canule de ponction (7), l'électrode de stimulateur cardiaque (5) peut être introduite dans le tissu cardiaque à travers le tuyau d'introduction (8) conduisant dans le tissu cardiaque depuis l'extérieur et dans le canal formé dedans par la canule de ponction (7).

4. Système d'électrode selon l'une des revendications 1 à 3, **caractérisé par le fait que** plusieurs ancres (9, 11, 12) sont prévues l'une derrière l'autre en direction axiale.

5. Système d'électrode selon l'une des revendications 1 à 4, **caractérisé par le fait qu'**il est conçu pour être biventriculaire et qu'une ligne d'alimentation (6) commune présente deux électrodes (5) se ramifiant et allant ensuite séparément au coeur (2), chacune ayant au moins une ancre servant à la fixation au coeur (2).

6. Système d'électrode selon l'une des revendications 1 à 5, **caractérisé par le fait que** l'anode (14) de l'électrode ou des électrodes (5) est disposée à l'extérieur du coeur (2) à distance de la cathode ou du pôle (3) se trouvant sur le coeur (2) et, dans le cas d'un système d'électrode ramifié biventriculaire, au niveau de la ligne d'alimentation (6) commune avant la ramification (13).
